# EUROPEAN PATENT SPECIFICATION

(11) **EP 1 113 772 B1**
(45) Date of publication and mention of the grant of the patent: **07.05.2003**
(21) Application number: 99968620.7
(22) Date of filing: 07.09.1999
(51) Int. Cl.: A61F 13/15

(54) **AIR PERMEABLE ABSORBENT ARTICLE HAVING A HYDROPHOBIC FOAM BACKSHEET LAYER**
LUFTDURCHLÄSSIGER ABSORBIERENDER ARTIKEL MIT ÄUSSERER SCHAUMSTOFFSCHICHT
ARTICLE ABSORBANT, PERMEABLE A L'AIR ET DOTE D'UNE COUCHE SUPPORT EN MOUSSE HYDROPHOBE

(30) Priority: 07.09.1998 EP 98116893
(43) Date of publication of application: 11.07.2001
(73) Proprietor: THE PROCTER & GAMBLE COMPANY, Cincinnati, Ohio 45202 (US)
(72) Inventor: CARLUCCI, Gianfranco, I-66100 Chieti (IT); D'INCECCO, Amadeo, Franco, I-65125 Pescara (IT); CIMINI, Carmine, I-65126 Pescara (IT); TORO, Carlo, I-65012 Cepagatti (IT)
(74) Representative: Hirsch, Uwe Thomas
(86) International application number: US9920502
(87) International publication number: WO00013637

(56) References cited:
- EP-A- 0 139 484
- EP-A- 0 557 677
- WO-A-97/19129
- WO-A-97/46186
- DE-A- 1 924 813
- GB-A- 2 305 863
- US-A- 3 805 790
- US-A- 3 993 074
- US-A- 4 639 254

## Description

### Field of the invention

The present invention relates to absorbent articles such as under arm sweat pads, bandages, sanitary napkins, panty liners, disposable diapers or adult incontinence products which are air permeable and comprise at least one layer of hydrophobic open celled foam.

### Background of the invention

Absorbent articles are known and they are made from several layers which are joined to each other. Generally an absorbent article comprises three main elements, namely a topsheet for liquid passage, a backsheet forming the outer surface, and an absorbent core interposed between the topsheet and the backsheet.

For example, in the case of a sanitary napkin, the absorbent product comprises typically three main components: a liquid pervious topsheet, a backsheet and an absorbent core. The absorbent core is enclosed by the backsheet and the topsheet and the product is worn such that the exposed surface of the topsheet faces the wearer of the absorbent product while the exposed surface of the backsheet faces the undergarment to which the product can be joint by a panty-fastening attachment means. Typically this is an adhesive but could also be a mechanical attachment.

For the purpose of improving the dryness, temperature sensation and comfort of such articles it is known to provide them with breathability. The prior art teaches the use of water vapor or air permeable backsheets. In particular it is known that air permeable backsheets should be hydrophobic to reduce probability of absorbent liquid leaching through the backsheet. Such air permeable backsheets are e.g. known from US 3,989,867, inventor Sisson, which is teaching to use an apertured formed film backsheet. Similar films, but primarily for use as topsheets, are known from EP-A- 18020, inventor Radel, or UK-A-1,526,778, inventor Thompson. Later disclosures include EP-A-710472 and EP-A-710471, inventor of both Depner, teaching to use multi layers hydrophobic structures for air permeability and reduced soil through.

All of these disclosures are genuine attempts to bridge the opposing needs to provide passage ways for air but passage ways for liquid - even under the pressure of squeezing such an article - should be prevented.

According to the present invention a new level of security and alternative to prior art systems against liquid soiling through the backsheet has been found while air permeability is maintained or even improved.

### Summary of the invention

The present invention relates to absorbent articles such as a sanitary napkin, panty liners, disposable diaper, incontinent product, under arm sweat pads, bandages etc. which are breathable and which have a hydrophobic open cell foam backsheet.

According to the present invention an air permeable disposable absorbent article conventionally comprising three layers is provided. The layers are an optional liquid permeable topsheet, an air permeable backsheet and an absorbent core between the topsheet and the backsheet.

Specifically, the present invention, having the features mentioned in the annexed claims, relates to absorbent articles having the backsheet with at least one layer of open cell foam which has permeability to air and water vapor but provides effectively a liquid barrier. Consequently all other layers of these breathable products also need to be permeable to air and water vapor to provide the desired benefits to the wearer / user of such articles.

The backsheet comprises a hydrophobic foam with open cells. Preferably the hydrophobic foam has a static water contact angle on it's outside surface of more than 90°, preferably more than 100° and most preferably more than 110°.

The foam can be made from a high internal phase emulsion. This usually produces a reticulated microporous foam structure. The emulsifier will typically be a surfactant in order to maintain the oil phase in suspension in the emulsion. According to one embodiment of the present invention the emulsifier in the high internal phase emulsion from which the foam is made is such that it does not deposit inside the foam cells. In an alternative embodiment the foam is washed after formation such that the emulsifier and other surfactant components within the foam cells are removed. In either case the desired hydrophobicity is provided by the inherent characteristics of the foam material as such.

In yet another embodiment the foam can also be treated - in its outer surface or also internally within the cells- to increase hydrophobicity such treatment can be achieved by fluorocarbon deposition or silicon oil coatings or other hydrophobic treatments.

According to the present invention the foam has preferably a thickness of less than 2 mm more preferably less than 1.4 mm. It is particularly desirable that the pore size of the foam cells is controlled such that the largest individual pore dimension is less than 65 % of the thickness of the foam in the backsheet, preferably it is less than 35% and most preferably it is less than 10% of the foam thickness.

In an alternative embodiment to providing the backsheet as a separate layer from the other components of the absorbent structure it would be a simplification for the manufacturing process of such articles if a single foam layer provides the backsheet and the absorbent core. When considering the thickness of the foam one portion on one side provides the backsheet and another portion of the foam on the other side provides at least part of the absorbent core. In such a construction the foam can be produced to be hydrophobic, hydrophilic or neutral with a treatment following the foam formation in which the backsheet portion is treated to be hydrophobic, the core portion is treated to be hydrophilic, or both portions are respectively treated to achieve a hydrophilic absorbent core portion and a hydrophobic backsheet portion f the foam.

The extend and details of the present invention are those which are found in the independent and dependent claims appended to the following detailed description of the components of the absorbent articles.

### Detailed description of the invention

For a better understanding of the present invention but without limitation the main components of a sanitary napkin as an example of an absorbent article will be now described. As stated before, typically such articles are of layered construction with each layer or group of layers and the article itself having a garment facing surface which is oriented to face in the direction of a garment during use of the article and a wearer facing surface facing in the opposite direction. Typically such articles comprise a liquid pervious topsheet layer forming the wearer facing surface of the article, an absorbent core layer and a backsheet layer forming the garment facing surface of the article. The absorbent core is interposed between the topsheet and the backsheet.

### Main Absorbent Article Components

### Topsheet

If present the topsheet layer is compliant, soft feeling, and non-irritating to the wearer's skin. The topsheet material also can be elastically stretchable in one or two directions. Further, the topsheet is inherently fluid pervious permitting fluids (e.g., menses and/or urine) to readily penetrate through its thickness. A suitable topsheet can be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers.

Preferred topsheets for use in the present are selected from high loft nonwoven topsheets and apertured formed film topsheets. Apertured formed films are especially preferred for the topsheet because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. 5,006,394. Particularly preferred microapertured formed film topsheets are disclosed in U.S. patent 4,609,518 and U.S. patent 4,629,643. The preferred topsheet for the present invention is the formed film described in one or more of the above patents and marketed on sanitary napkins by The Procter & Gamble Company of Cincinnati, Ohio as "DRI-WEAVE."

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The material forming the apertures of the formed film topsheet can be hydrophilic so as to help liquid to transfer through the topsheet faster than if the material was not hydrophilic. In a preferred embodiment, surfactant is incorporated into the polymeric materials of the formed film topsheet such as is described in U.S. Patent Application Serial No. 07/794,745, filed on November 19, 1991 or EP-A-166 058. Alternatively, the body surface of the topsheet can be made hydrophilic by treating it with a surfactant such as is described in U.S. 4,950,254.

### Absorbent core

The absorbent core typically includes the following components: (a) optionally a primary fluid distribution layer; (b) optionally, but preferably, a secondary fluid distribution layer; (c) a fluid storage layer; (d) optionally a fibrous ("dusting") layer underlying the storage layer; and (e) other optional components.

### a. Primary Fluid Distribution Layer

One optional component of the absorbent cores according to the present invention is the primary fluid distribution layer. This primary distribution layer typically underlies the topsheet and is in fluid communication therewith. The topsheet transfers the acquired menstrual fluid to this primary distribution layer for ultimate distribution to the storage layer. This transfer of fluid through the primary distribution layer occurs not only in the thickness, but also along the length and width directions of the absorbent product.

### b. Optional Secondary Fluid Distribution Layer

Also optional but a preferred component of the absorbent cores according to the present invention is a secondary fluid distribution layer. This secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized.

### c. Fluid Storage Layer

Positioned in fluid communication with, and typically underlying the primary or secondary distribution layers, is a fluid storage layer comprising certain absorbent gelling materials and/or other absorbent materials, which can form the carrier matrix for the absorbent gelling materials. Absorbent gelling materials are usually referred to as "hydrogels," "superabsorbent" "hydrocolloid" materials. Absorbent gelling materials are those materials that, upon contact with aqueous fluids, especially aqueous body fluids, imbibes such fluids and thus form hydrogels. These absorbent gelling materials are typically capable of absorbing large quantities of aqueous body fluids, and are further capable of retaining such absorbed fluids under moderate pressures. These absorbent gelling materials are typically in the form of discrete, nonfibrous particles.

The fluid storage layer can comprise solely absorbent gelling materials, or these absorbent gelling materials can be dispersed homogeneously or non-homogeneously in a suitable carrier or it can comprise solely an absorbent carrier material.

Suitable carriers include cellulose fibers, in the form of fluff, tissues or paper such as is conventionally utilized in absorbent cores. Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferred synthetic fibers have a denier of from about 3 denier per filament to about 25 denier per filament, more preferably from about 5 denier per filament to about 16 denier per filament. Also preferably, the fiber surfaces are hydrophilic or are treated to be hydrophilic. The storage layer can also include filler materials, such as Perlite, diatomaceous earth, Vermiculite, etc., that lower rewet problems.

If dispersed non-homogeneously in a carrier, the storage layer can be locally homogeneous, i.e. have a distribution gradient in one or several directions within the dimensions of the storage layer. Non-homogeneous distribution can also refer to laminates of carriers enclosing absorbent gelling materials partially or fully. If laminates are used they can be formed with or without absorbent gelling particles. In particular thermally bonded air laid fibrous sheets or laminates and/or thermally bonded wet laid sheets or laminates have been found useful, especially in the context of panty liners when no absorbent gelling material is used.

Preferably, the storage layer comprises from about 15 to 100% absorbent gelling materials and from 0 to about 85% carrier. More preferably, the storage layer comprises from about 30 to 100 %, most preferably from about 60 to 100% absorbent gelling materials and from 0 to about 70 %, most preferably from 0 to about 40 %, carrier.

Suitable absorbent gelling materials for use herein will most often comprise a substantially water-insoluble, slightly crosslinked, partially neutralized, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Patent 4,654,039 and reissued as RE 32,649. Preferred monomers include acrylic acid, methacrylic acid, and 2-acrylamido-2-methyl propane sulfonic acid. Acrylic acid itself is especially preferred for preparation of the polymeric gelling material. The polymeric component formed from the unsaturated, acid-containing monomers can be grafted onto other types of polymer moieties such as starch or cellulose. Polyacrylate grafted starch materials of this type are especially preferred. Preferred polymeric absorbent gelling materials that can be prepared from conventional types of monomers include hydrolyzed acrylonitrile grafted starch, polyacrylate grafted starch, polyacrylates, maleic anhydride-based copolymers and combinations thereof. Especially preferred are the polyacrylates and polyacrylate grafted starch.

While these absorbent gelling materials are typically in particle form, it is also contemplated that the absorbent gelling material can be in the form of macrostructures such as fibers, sheets or strips.

As an alternative or in addition to carriers with absorbent gelling material the use of hydrophilic open celled foams has been contemplated. Examples are disclosed in US 3,563,243 (Lindquist), US 4,554,297 (Dabi), US 4,740,520 (Garvey). Preferred foams are however hydrophilic foams made from High Internal Phase Emulsions (hereafter referred to as "HIPE"). See, for example, U.S. Patent 5,260,345 (DesMarais et al), issued December 7, 1993. These absorbent HIPE foams provide desirable fluid handling properties, including: (a) relatively good wicking and fluid distribution characteristics to transport the imbibed urine or other body fluid away from the initial impingement zone and into the unused balance of the foam structure to allow for subsequent gushes of fluid to be accommodated; and (b) a relatively high storage capacity with a relatively high fluid capacity under load, i.e. under compressive forces. These HIPE absorbent foams are also sufficiently flexible and soft so as to provide a high degree of comfort to the wearer of the absorbent article, and can be made relatively thin until subsequently wetted by the absorbent body fluid.

The nature and characteristics of HIPE absorbent foams are very much dependent on the type of components and the process conditions used to form the HIPE. This includes the emulsifier used in preparing the HIPE. The stability of HIPEs, and especially HIPEs having very high ratios of water phase to oil phase, depends in the emulsifier. The properties of the emulsifier used in making the HIPE can have other important effects on the fluid handling properties and characteristics of the resultant HIPE absorbent foam. The monomers used in making HIPE absorbent foams normally result in polymers that would be hydrophobic in the absence of a hydrophilizing surfactant. Even after the HIPE foam is washed and dewatered, some of the emulsifier remains within and on the surface of the foam. If it has the right surfactant properties, this residual emulsifier can hydrophilize this normally hydrophobic foam so as to make it capable of being wetted by, and thus absorbing, aqueous fluids.
Further improvements on the above mentioned HIPE foams have been documented in WO 96/21679, WO 96/21680, WO 96/21681, WO 96/21682, WO 97/07832, WO 98/00085.

### d. Optional Fibrous ("Dusting") Layer

An optional component for inclusion in the absorbent cores according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of laminates or of macrostructures such as fibers, sheets or strips, this fibrous "dusting" layer need not be included. However, because this "dusting" layer provides some additional fluid-handling capabilities such as rapid wicking of fluid along the length of the pad, its inclusion is typically preferred in absorbent cores according to the present invention.

### e. Other Optional Components

The absorbent cores according to the present invention can include other optional components normally present in absorbent webs. For example, a reinforcing scrim can be positioned within the respective layers, or between the respective layers, of the absorbent cores. Such reinforcing scrims should be of such configuration as to not form interfacial barriers to fluid transfer, especially if positioned between the respective layers of the absorbent core.

Another component which can be included in the absorbent core and preferably is provided close to or as part of the primary or secondary fluid distribution layer are odor control agents. These can be selected from active carbon or coated active carbon to conced the color, suitable zeolite or clay materials, or suitable zeolites and silicas are optionally incorporated in the absorbent core. Also absorbent gelling material in combination with certain zeolites and silicas have been found useful. These components can be incorporated in any desired form but often are included as discrete, non-fibrous particles.

### Backsheet

The backsheet layer prevents the exudates absorbed and contained in the absorbent core from wetting articles that contact the absorbent article such as pants, pyjamas and undergarments. The backsheet needs to be compliant and will readily conform to the general shape and contours of the human body. The backsheet preferably also can have characteristics allowing it to elastically stretch in one or two directions.

The backsheet according to the present invention provides breathability to the absorbent article by being water vapor and air permeable. The backsheet can be a laminate material i.e. it can comprise a combination with apertured film and/or non-woven material, and/or apertured formed film. Breathability if desired can be limited to the periphery or the center of the backsheet or it can be across the whole backsheet.

According to the present invention the backsheet comprises an open celled hydrophobic foam. Open celled hydrophobic foams are well known from the hydrophilic example mentioned above for use in the absorbent core. These examples can be directly utilized for making of the hydrophobic open celled foam of the backsheet of the present invention by leaving out any treatment to achieve hydrophilicity of the foam or introduce treatment to remove such hydrophilic treatment for example by washing with water, alcohol or oils. Most preferably hydrophobic foams are formed by use of hydrophobic monomeric materials in combination with hydrophobic additives. The conventional monomers for polyurethan foams are hence also useful in this alternative for providing hydrophobic foams.

As known from the HIPE foams mentioned above the emulsifier has a strong effect on the fluid handling characteristics of the HIPE foam. Hence it is desirable to use a emulsifier which does not easily deposit inside the foam structure or which is easily washed out of the foam structure by use of water and / or alcohol. An alternative would be the use of an emulsifier which has a very low surfactancy. This could of course be adverse to the ability to maintain the emulsion stable and would require other stability measures.

It is desirable that the hydrophobicity is at least such that a static water contact angle of more than 90°, preferably more than 100° and even better yet more than 110°, at least on that surface directed towards the outside of the backsheet, is achieved. A balance needs to be found between the soiling through performance requirement and the thickness of the foam in the backsheet. A too thick foam will not provide the desired flexibility and pliability for the article to be comfortable. It hence has been found that a foam thickness of less than 2 mm, preferably less than 1.4 mm can still provide the desired soil through performance and with reduced thickness increased air permeability while being acceptable from a comfort point of view in the context for example of sanitary napkins or under arm sweat pads.

A correlation between the pore size of the cells in the foam and the thickness of the film has also been found. Accordingly it is desirable that the largest pore dimension of the individual cells in the foam is less than the thickness of the film such that it stays below 65% of the thickness and preferably below 35% of the thickness. Particularly good results of prevention of soiling through have been found for foams having a largest pore dimension of less than 10% of the thickness of the foam in the backsheet.

The combination between a HIPE type foam layer in the core and a hydrophobic foam backsheet has been discussed above. The primary benefits in such an absorbent article construction are the simplicity for manufacturing such articles and the related economical and ecological improvements. The requirements for the backsheet and for the absorbent core as mentioned above remain the same whether the structure of core and backsheet is an integral structure such that they are made from a single foam substrate or whether they are made from separate substrates which are attached to each other.

### Joining of materials

It is important according to the present invention that the materials making up the absorbent article are not joined in such a way that the benefits from providing breathability in each individual component is eliminated by clogging the air exchange surfaces for example with adhesive. If necessary it is therefor desirable to provide adhesive in a discontinuous form between the materials which are reticulated and thereby provide almost no barrier to air and water vapor exchange. An alternative is to join the topsheet and the backsheet along their common periphery extending beyond the periphery of the absorbent core such that none of the area of the article co-extensive with the absorbent core has any reduced permeability due to construction adhesives.

When using hydrophobic foam with open cells in the backsheet of absorbent articles the overall construction of the article should be such that the structural liquid driving force ( often also referred to as capilarity ) and the surface material related driving force ( typically referred to as hydrophobicity / hydrophilicity due to material characteristics rather than structural characteristics) are balanced such that the storage layer in the absorbent core is the location within the article from which liquid has no natural tendency to escape. In physical terms the presence of liquid in the liquid storage layer of the absorbent core should be the status of highest entropy (lowest free energy) for migration of liquid within the article.

A particularly preferred absorbent article embodiment according to the present invention is a panty liner which comprises a formed film topsheet, an absorbent core with a laminate of two tissue layers with odour control material and absorbent gelling material between the layers and a hydrophobic foam backsheet made from a HIPE foam which was washed with water and alcohol.

## Claims

1. An air permeable absorbent article comprising two layers:
• an absorbent core,
• an air permeable backsheet positioned on a first side of said core, and
**characterized in that** said backsheet comprises a hydrophobic foam with open cells.

2. An absorbent article according to claim 1 further comprising a liquid permeable topsheet positioned on a second side of said core

3. An absorbent article according to claims 1, 2 **characterized in that** said hydrophobic foam has a static water contact angle of more than 100°, preferably more than 110°.

4. An absorbent article according to any of the preceding claims **characterized in that** said foam is made from a high internal phase emulsion in which the emulsifier is substantially not deposited inside the foam cells.

5. An absorbent article according to claim 1, 2 or 3 **characterized in that** said foam is made from a high internal phase emulsion in which the emulsifier is substantially washed out of the foam after formation of the foam.

6. An absorbent article according to any of the preceding claims **characterized in that** said foam has a thickness of less than 2 mm, preferably less than 1.4 mm.

7. An absorbent article according to any of the preceding claims **characterized in that** said foam has a pore size in which the largest pore dimension is less than 65 % preferably less than 35 % of the thickness of the foam.

8. An absorbent article according to any of the preceding claims **characterized in that** said absorbent core comprises a hydrophilic foam.

9. An absorbent article according to claim 8 **characterized in that** said absorbent core and said backsheet are integral comprising a backsheet portion and a core portion in which either said backsheet portion is treated to be hydrophobic or said core portion is treated to be hydrophilic or combinations thereof.

10. An absorbent article according to any of the preceding claims in which said article is a sanitary napkin, a panty liner, a disposable diaper, or an incontinent product.

## Patentansprüche

1. Luftdurchlässiger, absorbierender Artikel mit zwei Lagen:
- einem absorbierenden Kern,
- einer luftdurchlässigen Außenlage, die auf einer ersten Seite des Kerns positioniert ist, und
**dadurch gekennzeichnet, dass** die Außenlage einen hydrophoben Schaum mit offenen Zellen aufweist.

2. Absorbierender Artikel nach Anspruch 1, ferner umfassend eine flüssigkeitsdurchlässige Decklage, die auf einer zweiten Seite des Kerns positioniert ist.

3. Absorbierender Artikel nach den Ansprüchen 1, 2, **dadurch gekennzeichnet, dass** der hydrophobe Schaum einen statischen Wasser-Kontaktwinkel von mehr als 100°, vorzugsweise von mehr als 110° aufweist.

4. Absorbierender Artikel nach einem der vorherigen Ansprüche, **dadurch gekennzeichnet, dass** der Schaum aus einer starken Dispersum-Emulsion hergestellt ist, wobei der Emulgator im wesentlichen nicht innerhalb der Zellen des Schaums abgelagert ist.

5. Absorbierender Artikel nach Anspruch 1, 2 oder 3, **dadurch gekennzeichnet, dass** der Schaum aus einer starken Dispersum-Emulsion hergestellt ist, wobei der Emulgator im wesentlichen aus dem Schaum nach der Bildung des Schaums ausgewaschen wird.

6. Absorbierender Artikel nach einem der vorherigen Ansprüche, **dadurch gekennzeichnet, dass** der Schaum eine Dicke von weniger als 2 mm, vorzugsweise von weniger als 1,4 mm aufweist.

7. Absorbierender Artikel nach einem der vorherigen Ansprüche, **dadurch gekennzeichnet, dass** der Schaum eine Porengröße aufweist, wobei die größte Poren-Abmessung kleiner als 65 %, vorzugsweise kleiner als 35 % der Dicke des Schaums ist.

8. Absorbierender Artikel nach einem der vorherigen Ansprüche, **dadurch gekennzeichnet, dass** der absorbierende Kern einen hydrophilen Schaum aufweist.

9. Absorbierender Artikel nach Anspruch 8, **dadurch gekennzeichnet, dass** der absorbierende Kern und die Außenlage integral mit einem Außenlagen-Abschnitt und einem Kern-Abschnitt ausgebildet sind, wobei entweder der Außenlagen-Abschnitt behandelt ist, um hydrophob zu sein, oder der Kern-Abschnitt behandelt ist, um hydrophil zu sein, oder Kombinationen davon.

10. Absorbierender Artikel nach einem der vorherigen Ansprüche, wobei der Artikel eine Monatsbinde, ein Slip-Einsatz, eine Einweg-Windel oder ein Inkontinenz-Produkt ist.

## Revendications

1. Article absorbant perméable à l'air comprenant deux couches :
- une âme absorbante,
- une feuille de fond perméable à l'air placée sur un premier côté de ladite âme, et **caractérisé en ce que** ladite feuille de fond comprend une mousse hydrophobe à alvéoles ouvertes.

2. Article absorbant selon la revendication 1, comprenant, en outre, une feuille de dessus perméable aux liquides placée sur un deuxième côté de ladite âme.

3. Article absorbant selon les revendications 1, 2, **caractérisé en ce que** ladite mousse hydrophobe a un angle de contact statique avec l'eau supérieur à 100°, de préférence supérieur à 110°.

4. Article absorbant selon l'une quelconque des revendications précédentes, **caractérisé en ce que** ladite mousse est réalisée à partir d'une émulsion à phase interne élevée dans laquelle l'émulsifiant n'est pratiquement pas déposé à l'intérieur des alvéoles de la mousse.

5. Article absorbant selon la revendication 1, 2 ou 3, **caractérisé en ce que** ladite mousse est réalisée à partir d'une émulsion à phase interne élevée dans laquelle l'émulsifiant est pratiquement éliminé par lavage de la mousse après la formation de la mousse.

6. Article absorbant selon l'une quelconque des revendications précédentes, **caractérisé en ce que** ladite mousse a une épaisseur inférieure à 2 mm, de préférence inférieure à 1,4 mm.

7. Article absorbant selon l'une quelconque des revendications précédentes, **caractérisé en ce que** ladite mousse a une taille de pore dans laquelle la plus grande dimension de pore est inférieure à 65%, de préférence inférieure à 35%, de l'épaisseur de la mousse.

8. Article absorbant selon l'une quelconque des revendications précédentes, **caractérisé en ce que** ladite âme absorbante comprend une mousse hydrophile.

9. Article absorbant selon la revendication 8, **caractérisé en ce que** ladite âme absorbante et ladite feuille de fond sont solidaires, comprenant une partie de feuille de fond et une partie d'âme, dans lequel, soit ladite partie de feuille de fond est traitée de façon à être hydrophobe, soit ladite partie d'âme est traitée de façon à être hydrophile, soit ces deux parties sont combinées.

10. Article absorbant selon l'une quelconque des revendications précédentes, dans lequel ledit article est une serviette hygiénique, une garniture de culotte, une couche jetable ou un produit pour incontinent.
